# EUROPÄISCHE PATENTSCHRIFT

(11) **EP 1 348 383 B1**
(45) Veröffentlichungstag und Bekanntmachung des Hinweises auf die Patenterteilung: **04.05.2005**
(21) Anmeldenummer: 02006574.4
(22) Anmeldetag: 21.03.2002
(51) Int. Cl.: A61B 17/16, A61B 19/00, A61F 2/46, A61B 17/17

(54) **Retraktornavigation**
Retractor navigation device
APPAREIL DE NAVIGATION POUR UN ECARTEUR

(43) Veröffentlichungstag der Anmeldung: 01.10.2003
(73) Patentinhaber: BrainLAB AG, 85551 Kirchheim/Heimstetten (DE); Ritland, Stephen, Flagstaff, AZ 86001 (US)
(72) Erfinder: Ritland, Stephen, Dr., Flagstaff, AZ 86001 (US); Zeiss, Mario, 85586 Poing (DE); Brundobler, Matthias, 80992 München (DE)
(74) Vertreter: Schwabe - Sandmair - Marx

(56) Entgegenhaltungen:
- WO-A-99/29253
- DE-A- 10 005 880
- DE-A- 19 713 416
- US-B1- 6 351 659

## Beschreibung

Die vorliegende Erfindung betrifft eine Vorrichtung zur Unterstützung des Setzens von Pedikelschrauben. Solche Vorrichtungen werden auch "Retraktor" genannt, und sie dienen dazu, Pedikelschrauben in minimal invasiver Weise einzusetzen.

Die herkömmlichste Methode zum Setzen von Implantaten bzw. Implantationshilfsmitteln besteht darin, den gesamten Bereich über der in Frage kommenden Knochenstruktur, beispielsweise über einem Wirbel, mit Hilfe eines großen Hautschnittes freizulegen und danach die einzusetzenden Implantate, beispielsweise Schrauben, über vorher zu schneidende Gewindebohrungen einzubringen. Hierzu muss der behandelnde Chirurg große Erfahrung besitzen, Ärzte mit weniger Erfahrung könnten Strukturen anbohren, die unbedingt intakt gehalten werden müssen, also beispielsweise den Wirbelkanal eines Wirbelkörpers. Ein zusätzlicher Nachteil besteht auch darin, dass ein solcher Eingriff maximal invasiv ist. Mechanische Einbringhilfen, die weniger invasive Eingriffe ermöglichen sollen, beispielsweise Gelenkarm-Anordnungen, sind mechanisch äußerst kompliziert aufgebaute Geräte und ebenfalls nur mit großer Erfahrung korrekt handhabbar.

Wenn Anbohr- oder Einführhilfen verwendet werden, die eine Bohrung oder eine Durchführung aufweisen über die Implantate bzw. Implantationshilfsmittel gerichtet an die Knochenstruktur anzusetzen sind, hat sich auch die Verwendung von Navigationshilfen, zum Beispiel bekannten Navigations-Pointern, die durch das Einsetzen in eine solche Bohrung deren Ausrichtung in einem medizinischen Navigationssystem bestimmen sollen, als unzureichend erwiesen, da sich wegen der relativ großen Bohrung starke Winkelabweichungen ergeben können. Wegen der notwendigen Genauigkeit bei solchen Eingriffen sind solche Methoden grundsätzlich nicht geeignet.

Aus der DE 100 05 880 Al und aus der WO 99/292 53 sind Vorrichtungen bekannt, die einteilige Führungskörper für Instrumente aufweisen, die zur Behandlung von Wirbelkörpern dienen. In beiden Fällen ist der Führangskörper einteilig ausgestaltet und trägt Navigations-Markierungen, wodurch die Verwendung relativ unflexibel bleibt.

Aus der WO 02/060330 A1 ist eine Vorrichtung mit einem eintigen Innenteil bekannt. Die WO 02/060330 A1 ist Stand der Technik gemäß Art. 54(3) EPÜ.

Es ist die Aufgabe der vorliegenden Erfindung, eine flexibel einsetzbare Vorrichtung zur Unterstützung des Setzens von Pedikelschrauben bereitzustellen, die einen minimal invasiven Eingriff mit hoher Genauigkeit gestattet.

Diese Aufgabe wird erfindungsgemäß gelöst durch eine Vorrichtung zur Unterstützung des Setzens von Pedikelschrauben gemäß dem Anspruch 1. Die Unteransprüche definieren bevorzugte Ausführungsformen der Erfindung.

Die Begriffe "Innenteil" und "Außenteil" dienen im Rahmen der vorliegenden Beschreibung nicht unbedingt dazu, ein Innen-Außen-Positionsverhältnis dieser Teile zu definieren. Es soll lediglich angedeutet werden, dass das "Außenteil" dasjenige Teil der Vorrichtung ist, welches als eine Führung dient, in welcher das "Innenteil" richtungsbestimmt bewegt und positioniert werden kann.

Medizinische Navigationssysteme, wie sie im Rahmen der vorliegenden Erfindung grundsätzlich verwendet werden könnten, sind beispielsweise aus der DE 196 39 615 C2 oder aus der US 6,285,902 bekannt. Dadurch, dass eine erfindungsgemäße Vorrichtung mittels ihrer Referenzeinrichtung im medizinischen Navigationssystem navigiert werden kann, kann das Setzen von Implantaten oder die Verwendung von Implantationshilfsmitteln mit der Vorrichtung sehr genau und zielgerichtet sowie kontrollierbar durchgeführt werden, was es wiederum möglich macht, die entsprechenden Eingriffe in minimal invasiver Weise durchzuführen. Traumata am Weichgewebe vor dem zu behandelnden Knochen können auf ein Minimum reduziert werden. Es ist nicht mehr nötig, andere und eventuell auch ungenaue Hilfsmittel wie mechanische Einbringhilfen oder Navigations-Pointer zu verwenden, und ein Chirurg kann den Eintrittspunkt, Eintrittswinkel und die Größe von Pedikelschrauben vorab so genau planen, dass unerwünschte Schädigungen an Patientenkörperteilen vermieden werden können.

Ein weiterer Vorteil der erfindungsgemäßen Vorrichtung besteht darin, dass ein Echtzeit-Tracking der Vorrichtung möglich wird, wodurch die früher oft verwendeten und zwischenzeitlich aufgenommen Fluoroskopie-Aufnahmen nunmehr lediglich als ergänzendes Navigations-Hilfsmittel benötigt werden und verwendet werden können. Die Fluoroskopie, die als alleiniges Navigationsinstrument relativ ungeeignet war, da ständig neue Röntgenaufnahmen erstellt werden mussten, wird nunmehr zu einem geeigneten Ergänzungsinstrument für die Navigation, um beispielsweise Knochenstrukturen in den Schichtbildaufnahmen des Navigationssystems besser sichtbar zu machen.

Gemäß einer Ausführungsform der vorliegenden Erfindung ist die Referenzeinrichtung der Vorrichtung am Innenteil angeordnet. In diesem Fall kann das Innenteil ständig navigationsüberwacht bleiben, und zwar auch dann, wenn es nach dem Setzvorgang vom Außenteil getrennt wird, also wenn beispielsweise das Innenteil von dem Außenteil abgezogen wird, nachdem dieses vorpositioniert worden ist. Dies ermöglicht es dem Chirurgen, mehrere, nicht navigierte Außenteile zu verwenden. Dabei können gleiche Arbeitsschritte an verschiedenen Körperteilen vorab durchgeführt werden, zum Beispiel können mehrere Außenteile in ihre Position gesetzt werden, um dann einem navigierten Innenteil jeweils den Zugang zu seiner Ansetzstelle zu ermöglichen. Das Innenteil wird dann für jeweils denselben Arbeitsschritt aus dem einen Außenteil entnommen und in das nächste Außenteil eingeführt.

Bei einer weiteren Ausführungsform der vorliegenden Erfindung ist die Referenzeinrichtung der Vorrichtung am Außenteil angeordnet. Eine solche Ausgestaltung ist insbesondere dann von Vorteil, wenn das Außenteil zusammen mit dem Innenteil nur einmal angesetzt werden muss und alle mit Hilfe der Vorrichtung durchzuführenden Eingriffe an einer Stelle nacheinander erledigt werden.

Natürlich ist es gemäß der vorliegenden Erfindung ebenso möglich, sowohl am Außenteil als auch am Innenteil eine Referenzeinrichtung anzuordnen. Es besteht dann zum Beispiel die Möglichkeit, zunächst ein Außenteil zum gewünschten Behandlungspunkt hin einzuführen, und damit eine Vorabpositionierung für die spätere Einführung des Innenteils durchzuführen. Die Navigation kann dann auf der Basis des navigierten Innenteils weitergeführt werden, solange die eigentliche Behandlung dauert, da dieses exakt am vorbestimmten Ansetzpunkt der Knochenstruktur angeordnet werden kann. Wenn für Innenteil und Außenteil jeweils charakteristische Referenzeinrichtungen verwendet werden, stellt die Unterscheidung dieser beiden Vorrichtungsteile bei der Navigation kein Problem dar.

Gemäß der Erfindung werden bei der Vorrichtung mehrere Innenteile bereitgestellt, die unterschiedliche große Durchführungen für Implantate bzw. Implantationshilfsmitteln mit unterschiedlichen Abmessungen aufweisen. Damit wird es möglich, die jeweils benutzten Instrumente (zum Beispiel Fräser, Gewindeschneidvorrichtung, Implantat), genau und in exakter äußerer Anlage durch die jeweilige Durchführung des gerade benutzten Innenteils zu führen.

Am Innenteil und/oder am Außenteil kann vorteilhafterweise eine Setzspitze vorgesehen sein, insbesondere eine solche, deren Form so angepasst ist, dass sie in definierter Lage in eine passende Kalibrierungsaufnahme eines Kalibrierungsinstruments eingesetzt werden kann. Ein solches Kalibrierungsinstrument dient dazu, dem Navigationssystem zu einem Zeitpunkt vor der Verwendung der Vorrichtung genau deren Position und Ausrichtung mitzuteilen. Dies ist dann wichtig, wenn beispielsweise abnehmbare Referenzeinrichtungen verwendet werden, die an beliebig ausgestaltete Vorrichtungen angesetzt werden können. Falls Vorrichtungen mit Innen- und Außenteilen Verwendung finden, die standardisiert sind, genügt es dem Navigationssystem vorher den Vorrichtungstyp mitzuteilen, wodurch die Notwendigkeit einer Kalibrierung entfällt.

Um das Innenteil gut handhabbar zu machen, kann ein Griffabschnitt an ihm angeordnet werden.

Je nach Anwendungsfall kann die erfindungsgemäße Vorrichtung aus verschiedenen Materialien hergestellt werden. Das Innenteil und das Außenteil können aus Metall gefertigt sein, insbesondere aus Stahl (Chirurgenstahl). Wenn ein Metall (Stahl) zur Herstellung der Vorrichtungsteile verwendet wird, eignet sich als Referenzeinrichtung eine mit einem optisch basierten Navigationssystem verfolgbare Referenzeinrichtung, insbesondere eine Reflektorenoder Emitteranordnung.

Andererseits besteht die Möglichkeit, das Innenteil und/oder das Außenteil aus Kunststoff, insbesondere aus Kevlar herzustellen. Dies ist speziell dann von Vorteil, wenn die Referenzeinrichtung eine mit einem magnetisch basierten Navigationssystem verfolgbare Referenzeinrichtung, insbesondere eine Miniaturspulenanordnung ist.

Aus Obigem geht schon hervor, dass die erfindungsgemäße Vorrichtung grundsätzlich zwar mit einem Navigationssystem arbeitet, der Verwender jedoch in der Auswahl des Navigationssystems relativ frei ist.

Gemäß einer weiteren bevorzugten Ausführungsform der vorliegenden Erfindung sind das Innenteil und/oder das Außenteil aus einem für Röntgenstrahlung durchlässigen Material hergestellt, wodurch sichergestellt werden kann, dass die Instrumente keine Sichtschatten auf während der Behandlung erstellten Röntgenaufnahmen werfen, die Teile der Strukturen verdecken, die sichtbar gemacht werden sollen.

Die Erfindung wird im Weiteren anhand von Ausführungsbeispielen näher beschrieben. In den Zeichnungen zeigen:
- Figur 1: eine erfindungsgemäße Vorrichtung (Retraktor) mit einer Referenzeinrichtung am Innenteil; und
- Figur 2: eine erfindungsgemäße Vorrichtung (Retraktor) mit einer Referenzeinrichtung am Außenteil.

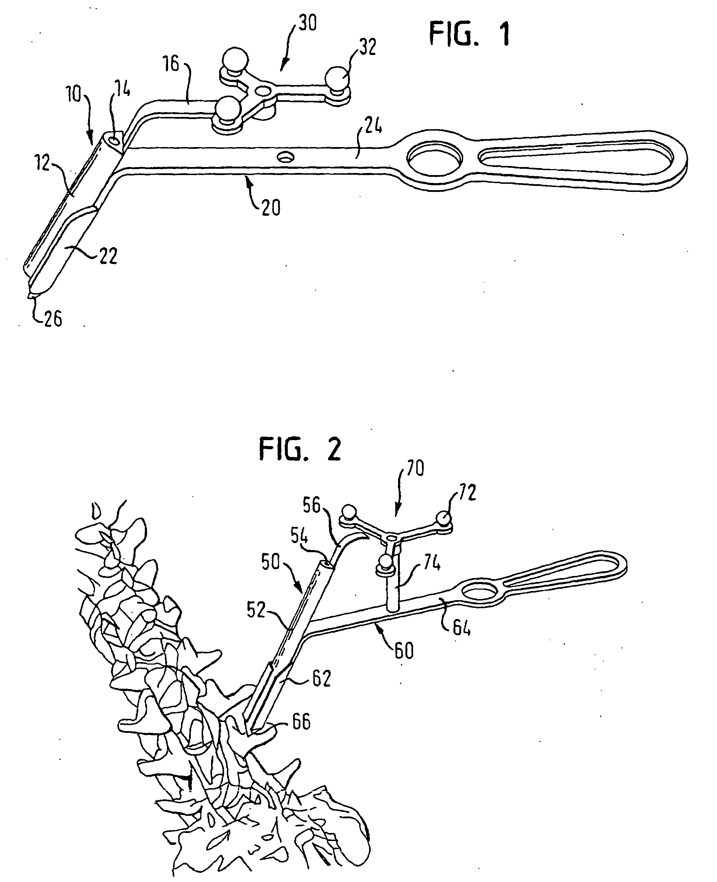

Die in Figur 1 dargestellte, erfindungsgemäße Vorrichtung weist ein Innenteil 10 und ein Außenteil 20 auf. Im Führungsabschnitt 22 des Außenteils 20 wird der Führungseingriffsabschnitt 12 des Innenteils 10 geführt, wobei der Führungsabschnitt 22 eine Art Schiene für den Führungseingriffsabschnitt 12 bildet, die den unteren Teil des dreieckförmigen Führungseingriffsabschnittes 12 nach Art einer Längsführung in Eingriff nimmt.

Im Führungseingriffsabschnitt 12 befindet sich eine durchgehende Längsbohrung bzw. Durchführung 14 durch die Instrumente wie Fräser oder Gewindeschneideinrichtungen und zum Beispiel auch Implantate geführt in eine Knochenstruktur eingebracht werden können. Über die Verlängerung 16, die auch als Griffstück für das Innenteil 10 verwendet werden kann, ist am Innenteil 10 bzw. am Führungseingriffsabschnitt 12 eine Referenzeinrichtung 30 angebracht, die aus einer Anordnung dreier Reflektoren 32 besteht, deren Position von einem Navigationssystem erfasst werden kann. Die Reflektoren 32 des Navigationssterns 30 besitzen eine für das Innenteil 10 charakteristische Anordnung und gestatten somit dessen Positionsverfolgung zu jedem Zeitpunkt.

Wie schon vorher erwähnt, wird der Führungseingriffsabschnitt 12 des Innenteils 10 im Führungsabschnitt 22 des Außenteils 20 schienenartig geführt. Das Außenteil 20 weist am vorderen Ende des Führungsabschnittes 22 eine Setzspitze 26 auf, mit der das Außenteil exakt an den vorbestimmten Ansetzpunkt an der Knochenstruktur angesetzt werden kann. Grundsätzlich kann eine solche Setzspitze auch am Innenteil, d. h. an dessen vorderster Spitze vorgesehen sein.

An den Führungsabschnitt 22 schließt sich ein Halteabschnitt 24 an, den der Chirurg bei der Verwendung der Vorrichtung optimal greifen kann.

Die in Figur 2 dargestellte Vorrichtung unterscheidet sich von derjenigen aus Figur 1 darin, dass die Referenzeinrichtung, also der Referenzstern 70 nicht am Innenteil 50, sondern am Außenteil 60, nämlich dort am Halteabschnitt 64 angebracht ist. Dies geschieht mittels der Befestigungsstange 74, die an ihrem oberen Ende den Referenzstern 70 mit seinen Reflektoren 72 trägt. Ansonsten weist auch der Außenteil 60 der Vorrichtung nach Figur 2 wieder einen Halteabschnitt 64, einen Führungsabschnitt 62 und eine Setzspitze 66 auf, während der Innenteil 50 wiederum einen Führungseingriffsabschnitt 52 mit einer Durchführung 54, also einer Durchgangsbohrung 54 umfasst. In diesem Fall ist die an den Führungseingriffsabschnitt 52 anschließende Verlängerung lediglich als ein Griffstück 56 vorgesehen, mit dem das Innenteil 50 gehandhabt werden kann. Die Figur 2 zeigt, wie die erfindungsgemäße Vorrichtung gemäß der zweiten Ausführungsform an einem Wirbel angesetzt werden kann.

Im Weiteren wird nunmehr zur Erläuterung der Erfindung noch die Verwendung der Vorrichtung im Rahmen einer Operation beschrieben, bei der Pedikelschrauben in Wirbel eingebracht und außenseitig mit Stäben verbunden werden.

Zunächst wird mittig über den zu behandelnden Wirbeln ein kurzer Hautschnitt gemacht, der nur wenige Zentimeter lang sein muss, also sehr viel kürzer sein kann, als bei herkömmlichen "offenen Operationen". Der Schnitt wird bis zu den Muskelpaketen hin vertieft und danach wird die Haut geöffnet, welche die Muskelpakete umgibt. Das Pedikel, d. h. der Bereich, in den die Schraube eingebracht werden soll, wird erfühlt und die erfindungsgemäße Vorrichtung mit Außenteil und Innenteil wird zwischen den Muskelpaketen eingebracht, bis die Setzspitze exakt an der Position liegt, wo die Schrauben eintreten sollen. Danach wird mittels des Navigationssystems die Eindringposition kontrolliert und gegebenenfalls korrigiert, und die Eindringtraektorie wird geplant. Weil der erfindungsgemäße Retraktor über die Referenzeinrichtung navigiert werden kann, kann beispielsweise die Traektorie aus verschiedenen Winkeln im Navigationssystem berechnet und auf einem Bildschirm angezeigt werden, und die Position des Retraktors wird durch das Ausrichten des Außenteils solange korrigiert, bis ein geeigneter Eindringwinkel sichergestellt ist.

Nunmehr kann mittels eines Fräsers durch die Bohrung im Führungseingriffsabschnitt des Innenteils der Knochen an der Eintrittstelle geöffnet werden. Der Fräser wird dann aus der Durchführung des Innenteils wieder entfernt und danach wird ein Gewindeschneider eingesetzt, mit Hilfe dessen das Gewinde in dem Pedikel eingebracht wird.

Nach dem Entfernen des Gewindeschneiders kann mittels eines Pedikelprüfers überprüft werden, ob eine Perforation des Pedikels zur Innenwand hin vorliegt, was bei einer geeigneten Navigation in keinem Falle vorkommen sollte.

Nunmehr kann das Innenteil der erfindungsgemäßen Vorrichtung entfernt und die Schraube kann am Führungsabschnitt des Außenteils entlang in das vorbereitete Gewinde eingebracht werden.

Dieser Vorgang kann für mehrere Schrauben an übereinander liegenden Wirbeln, die nicht unbedingt aneinander angrenzen müssen, wiederholt werden, bis eine ausreichende Anzahl von Schrauben eingesetzt sind, die dann mit Stäben verbunden werden. Wie schon oben beschrieben, besteht natürlich auch die Möglichkeit, die oben beschriebene Reihenfolge zu ändern. Insbesondere können einige gleiche Arbeitsschritte an verschiedenen Wirbeln unmittelbar nacheinander durchgeführt werden, wonach eine zweite Arbeitsschritt-Gruppe folgt. Natürlich lassen sich auf diese Weise auch Flüssigimplantate (bone craft) einbringen, wobei die erfindungsgemäße Vorrichtung dann als Führung eine Kanüle verwendet wird.

## Patentansprüche

1. Vorrichtung zur Unterstützung des Setzens von Pedikelschrauben, umfassend
- ein Außenteil (20, 60), das einen Halteabschnitt (24, 64) und einen Führungsabschnitt (22, 62) aufweist, und
- mehrere Innenteile (10, 50), die jeweils einen Abschnitt (12, 52), der im Führungsabschnitt geführt wird, und einer unterschiedlich große Durchführungen für Pedikelschrauben oder Implantationshilfsmitteln mit unterschiedlichen Abmessungen aufweisen, und
- einer Referenzeinrichtung (30, 70) zur Navigation der Vorrichtung mittels eines medizinischen Navigationssystems, die am Außenteil (20, 60) und/oder am jeweiligen Innenteil (10, 30) angeordnet ist, wodurch das Setzen der Pedikelschraube navigationsgestützt erfolgen kann.

2. Vorrichtung nach Anspruch 1, **dadurch gekennzeichnet, dass** die Referenzeinrichtung (30) am jeweiligen Innenteil (10) angeordnet ist.

3. Vorrichtung nach Anspruch 1, **dadurch gekennzeichnet, dass** die Referenzeinrichtung (70) am Außenteil (60) angeordnet ist.

4. Vorrichtung nach Anspruch 1, **dadurch gekennzeichnet, dass** am jeweiligen Innenteil (10, 50) und am Außenteil (20, 60) eine Referenzeinrichtung (30, 70) angeordnet ist.

5. Vorrichtung nach einem der Ansprüche 1 bis 4, **dadurch gekennzeichnet, dass** am jeweiligen Innenteil und/ oder am Außenteil eine Setzspitze (26, 66) vorgesehen ist.

6. Vorrichtung nach einem der Ansprüche 1 bis 5, **dadurch gekennzeichnet, dass** an dem jeweiligen Innenteil ein Griffabschnitt (16, 56) angeordnet ist.

7. Vorrichtung nach einem der Ansprüche 1 bis 6, **dadurch gekennzeichnet, dass** das jeweilige Innenteil (10, 50) und/oder das Außenteil aus Metall, insbesondere Stahl hergestellt sind.

8. Vorrichtung nach Anspruch 7, **dadurch gekennzeichnet, dass** die Referenzeinrichtung eine mit einem optisch basierten Navigationssystem verfolgbare Referenzeinrichtung, insbesondere eine Reflektoren- oder Emitteranordnung ist.

9. Vorrichtung nach einem der Ansprüche 1 bis 6, **dadurch gekennzeichnet, dass** das jeweilige Innenteil (10, 50) und/oder das Außenteil aus Kunststoff, insbesondere aus Kevlar hergestellt sind.

10. Vorrichtung nach Anspruch 9, **dadurch gekennzeichnet, dass** die Referenzeinrichtung eine mit einem magnetisch basierten Navigationssystem verfolgbare Referenzeinrichtung, insbesondere eine Miniaturspulenanordnung ist.

11. Vorrichtung nach Anspruch 9 oder 10, **dadurch gekennzeichnet, dass** das jeweilige Innenteil (10, 50) und/oder das Außenteil aus einem für Röntgenstrahlung durchlässigen Material hergestellt sind.

## Claims

1. A device for assisting in positioning pedical screws, comprising:
- an outer part (20, 60) comprising a supporting section (24, 64) and a guiding section (22, 62); and
- a number of inner parts (10, 50), each comprising a section (12, 52) guided in the guiding section and passages of different sizes for pedical screws or implanting aids having different dimensions; and
- a reference means (30, 70) for navigating said device by means of a medical navigation system, which is arranged on said outer part (20, 60) and/or on said respective inner part (10, 50), whereby said pedical screw can be positioned with navigational assistance.

2. The device as set forth in claim 1, **characterised in that** said reference means (30) is arranged on said respective inner part (10).

3. The device as set forth in claim 1, **characterised in that** said reference means (70) is arranged on said outer part (60).

4. The device as set forth in claim 1, **characterised in that** a reference means (30, 70) is arranged on said respective inner part (10, 50) and on said outer part (20, 60).

5. The device as set forth in any one of claims 1 to 4, **characterised in that** a positioning tip (26, 66) is provided on said respective inner part and/or on said outer part.

6. The device as set forth in any one of claims 1 to 5, **characterised in that** a grip section (16, 56) is arranged on said respective inner part.

7. The device as set forth in any one of claims 1 to 6, **characterised in that** said respective inner part (10, 50) and/or said outer part are made of metal, in particular of steel.

8. The device as set forth in claim 7, **characterised in that** said reference means is a reference means which may be tracked using an optically based navigation system, in particular an arrangement of reflectors or emitters.

9. The device as set forth in any one of claims 1 to 6, **characterised in that** said respective inner part (10, 50) and/or said outer part are made of plastic, in particular of Kevlar.

10. The device as set forth in claim 9, **characterised in that** said reference means is a reference means which may be tracked using a magnetically based navigation system, in particular an arrangement of miniature coils.

11. The device as set forth in claim 9 or 10, **characterised in that** said respective inner part (10, 50) and/or said outer part are made of a material which is permeable to x-rays.

## Revendications

1. Dispositif d'assistance pour la pose de vis de pédicule, comprenant
- une pièce extérieure (20, 60), qui présente une partie de prise (24, 64) et une partie de guidage (22, 62),
- plusieurs pièces intérieures (10, 50), qui présentent chacune une partie (12, 52), qui est guidée dans la partie de guidage, et des passages de grandeur différente pour des vis de pédicule ou des accessoires d'implants de dimensions différentes, et
- un dispositif de référence (30, 70) pour guider la navigation du dispositif au moyen d'un système de navigation médical, qui est disposé sur la pièce extérieure (20, 60) et/ou sur la pièce intérieure respective (10, 30), par lequel la pose de la vis de pédicule peut être effectuée de façon assistée par navigation.

2. Dispositif selon la revendication 1, **caractérisé en ce que** le dispositif de référence (30) est disposé sur la pièce intérieure respective (10).

3. Dispositif selon la revendication 1, **caractérisé en ce que** le dispositif de référence (70) est disposé sur la pièce extérieure (60).

4. Dispositif selon la revendication 1, **caractérisé en ce qu'**un dispositif de référence (30, 70) est disposé sur la pièce intérieure respective (10, 50) et sur la pièce extérieure (20, 60).

5. Dispositif selon l'une quelconque des revendications 1 à 4, **caractérisé en ce qu'**il est prévu une pointe de pose (26, 66) sur la pièce intérieure respective et/ou sur la pièce extérieure.

6. Dispositif selon l'une quelconque des revendications 1 à 5, **caractérisé en ce qu'**une partie de saisie (16, 56) est disposée sur la pièce intérieure respective.

7. Dispositif selon l'une quelconque des revendications 1 à 6, **caractérisé en ce que** la pièce intérieure respective ((10, 50) et/ou la pièce extérieure est fabriquée en métal, notamment en acier.

8. Dispositif selon la revendication 7, **caractérisé en ce que** le dispositif de référence est un dispositif de référence pouvant être suivi par un système de navigation à base optique, notamment un agencement de réflecteurs ou d'émetteurs.

9. Dispositif selon l'une quelconque des revendications 1 à 6, **caractérisé en ce que** la pièce intérieure respective (10, 50) et/ou la pièce extérieure est fabriquée en matière plastique, notamment en Kevlar.

10. Dispositif selon la revendication 9, **caractérisé en ce que** le dispositif de référence est un dispositif de référence pouvant être suivi par un système de navigation à base magnétique, notamment un agencement de bobines miniatures.

11. Dispositif selon la revendication 9 ou 10, **caractérisé en ce que** la pièce intérieure respective (10, 50) et/ou la pièce extérieure est fabriquée en une matière perméable au rayonnement X.
